# EUROPEAN PATENT APPLICATION

(11) **EP 2 428 213 A1**
(43) Date of publication of application: **14.03.2012**
(21) Application number: 11179750.2
(22) Date of filing: 21.07.2006
(51) Int. Cl.: A61K 33/00, A61K 31/7064, A61P 35/00

(54) **STABILIZED CHLORITE SOLUTIONS IN COMBINATION WITH FLUOROPYRIMIDINES FOR USE IN CANCER TREATMENT**

(30) Priority: 21.07.2005 US 701075 P
(62) Divisional of application: 06761149.1
(71) Applicant: Nuvo Research AG, 1700 Fribourg (CH)
(72) Inventor: Meuer, Stefan, D-69121 Heidelberg (DE); Giese, Thomas, D-69198 Schriesheim (DE); Kuhne, Frederich-Willhelm, D-39164 Wanzleben (DE)
(74) Representative: Brand, Thomas Louis

(57) **Abstract**

Pharmaceutical compositions for treating neoplastic disorders and uses thereof are provided. Said pharmaceutical compositions comprise stabilised chlorite solutions (eg, WFIO) and a fluoropyrimidine, 5-FU, or a 5-FU prodrug (eg, capecitabine, doxifluridine, UFT, S-1, or BOF-A2). The use of the above stabilised chlorite solution in combination with a fluoropyrimidine dramatically improves the quality of life index (QOL) of a patent undergoing cancer chemotherapy. Cancers that can be treated include cancers of the pancreas, gastrointestinal, head, neck and breast.

## Description

### FIELD OF THE INVENTION

The present invention releates to a method for treatment of cancer. The method uses a 5-fluorouracil (5-FU) drug or a 5-FU prodrug in combination with a stabilized chlorite solution to treat a variety of cancers.

### DESCRIPTION OF THE PRIOR ART

5-Fluorouracil (5-FU) is a commonly prescribed anticancer drug, displaying significant activity against cancers of the gastrointestinal tract, head and neck and breast. 5-FU is a pyrimidine analog that is administered intravenously.

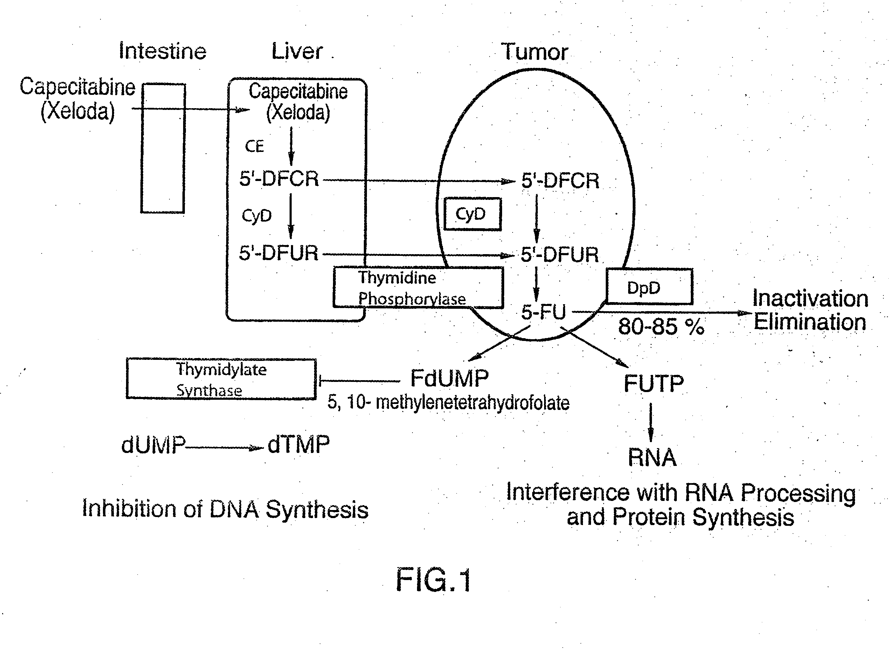

Capecitabine (XELODA®) is a new fluropyrimidine prodrug which can be administered orally. While not wishing to be bound by any particular theory or mode of action, it is believed that capecitabine is preferentially converted to the cytotoxic moiety 5-FU in target tissue through a series of three metabolic steps as shown in Figure 1. See, for example, (i) Miwa M, Design of a novel oral fluoropyrimidine carbamate, capacitabene, which generates 5-fluorouracil selectively in tumours concentrated in human liver and cancer tissue. Eur J Cancer 1998;34:1274-1281, and (ii) United States patent 4,966,891 [Fujiu et al.]. Although capecitabine represents a significant improvement in conventional 5-FU treatments, it is not without significant adverse effects and patients still often endure a low quality of life while taking the drug.

Pancreatic cancer in particular is a devastating illness characterized by significant morbidity and a brief median survival. For example in 2004, 31,860 people were diagnosed with pancreatic cancer in the United States -- 31,270 died from the disease. (See Jemel A,: Cancer Statistics, 2004. CA Cancer J Clin 2004, 54:8-29).

Currently available treatments used in pancreatic cancer have little impact on median survival. For instance, in randomized phase III trials in pancreatic cancer, the standard of care treatment gemcitabine consistently shows a median survival of 5 to 6 months. (See Friberg G & Kinder. Chemotherapy for Advanced Pancreatic Cancer: Past, Present, and Future, Current Oncology Reports, 7:186-195).

Phase II studies with 5-FU show median survival between show median survival of about 4 to 6 months. See Crown J, Lack of efficacy of high dose leucovorin and fluorouracil in patients with advanced pancreatic adenocarcinoma; J Clin Oncol 1991; 9:1682-1686; and Decaprio JA, Fluorouracil and high dose leucovorin in previously untreated patients with advanced adenocarcinoma of the pancreas: results of a phase II trial. J Clin Oncol 1991;9:1682-1686).

Median survival of patients in a phase II study of capecitabine was 182 days. See Cartright et al, Phase II study of oral capacitabine in patients with advanced or metastatic pancreatic cancer. J Clin Oncol 2002; 20:160-164.

It is apparent, therefore, that improved treatment regimens that reduce the frequency of dosing, that improve the efficacy of 5-FU or a 5-FU prodrug, and that improve patient quality of life are greatly to be desired. There is also a significant need for improvement treatments for pancreatic cancer patients.

### SUMMARY OF THE INVENTION

It is an object of the present invention to obviate or mitigate at least one of the above-mentioned disadvantages of the prior art.

It is another object of the present invention to provide a novel method for treating a neoplastic disorder in a patient.

It is a further object of the present invention to provide a novel method of enhancing the efficacy of a therapeutic agent in a subject being treated with the therapeutic agent.

Accordingly, in one of its aspects, the present invention provides a method of treating a neoplastic disorder in a patient, comprising administering to the patient an effective amount of a combination of a cytotoxic agent and a stabilized chlorite solution, wherein the cytotoxic agent comprises 5-FU or a compound that is converted to 5-FU in the body of the patient.

In another of its aspects, the present invention provides a method of enhancing the efficacy of a therapeutic agent in a subject being treated with the therapeutic agent, comprising administering to the patient an effective amount of a stabilized chlorite solution, wherein the therapeutic agent is degraded or inactivated by an enzyme in the subject, and wherein the stabilized chlorite solution inhibits the expression or activity of the enzyme.

In yet another of its aspects, the present invention provides a pharmaceutical composition comprising a cytotoxic agent and a stabilized chlorite solution, wherein the cytotoxic agent comprises 5-FU or a compound that is converted in vivo to 5-FU.

In yet another of its aspects, the present invention provides a neoplastic disorder treatment composition comprising a cytotoxic agent and a stabilized chlorite solution, wherein the cytotoxic agent comprises 5-FU or a compound that is converted in vivo to 5-FU.

In yet another of its aspects, the present invention provides use of a cytotoxic agent and a stabilized chlorite solution for treatment of a neoplastic disorder, wherein the cytotoxic agent comprises 5-FU or a compound that is converted in vivo to 5-FU.

In yet another of its aspects, the present invention provides use of a cytotoxic agent and a stabilized chlorite solution for preparation of a pharmaceutical composition for treatment of a neoplastic disorder, wherein the cytotoxic agent comprises 5-FU or a compound that is converted in vivo to 5-FU.

### BRIEF DESCRIPTION OF THE DRAWINGS

Embodiments of the present invention will be described with reference to the accompanying drawings, wherein like reference numerals denote like parts, and in which:
Figure 1 illustrates the steps by which capecitabine is believed to be converted into 5-FU in vivo;
Figure 2 illustrates the modulation of gene expression by WF10 in vivo; and

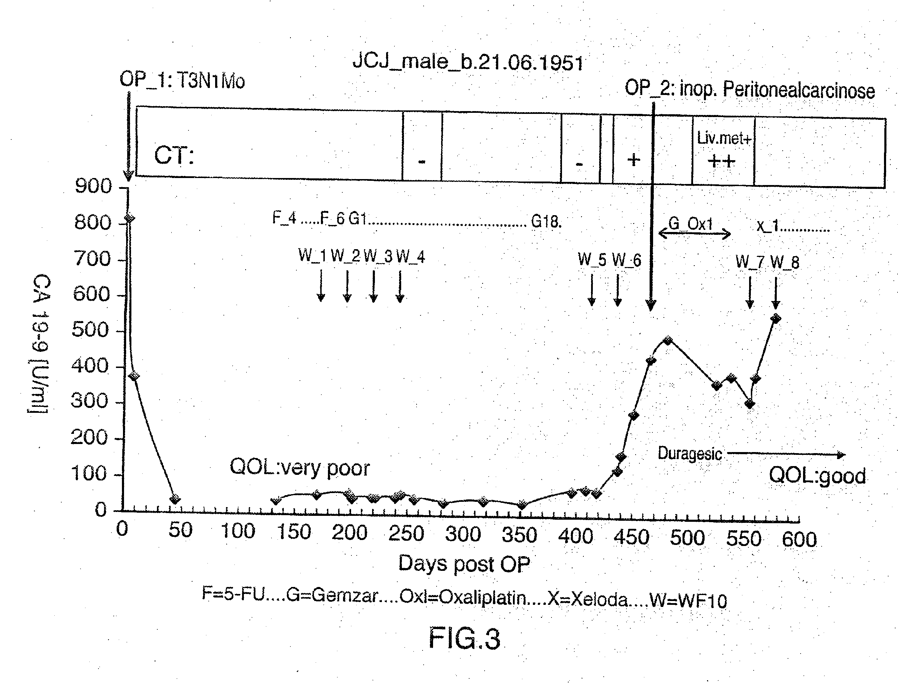

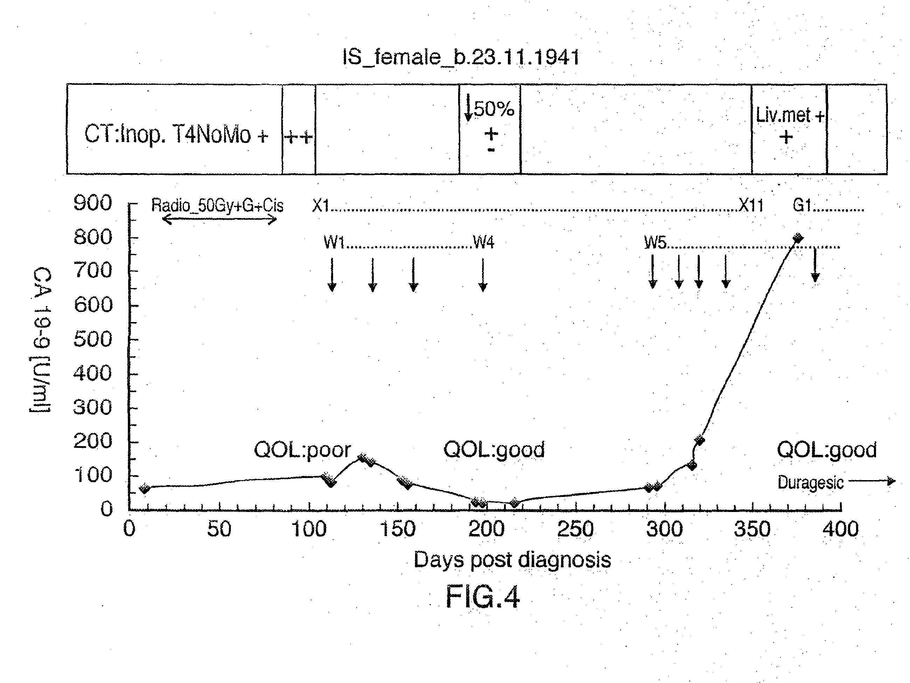

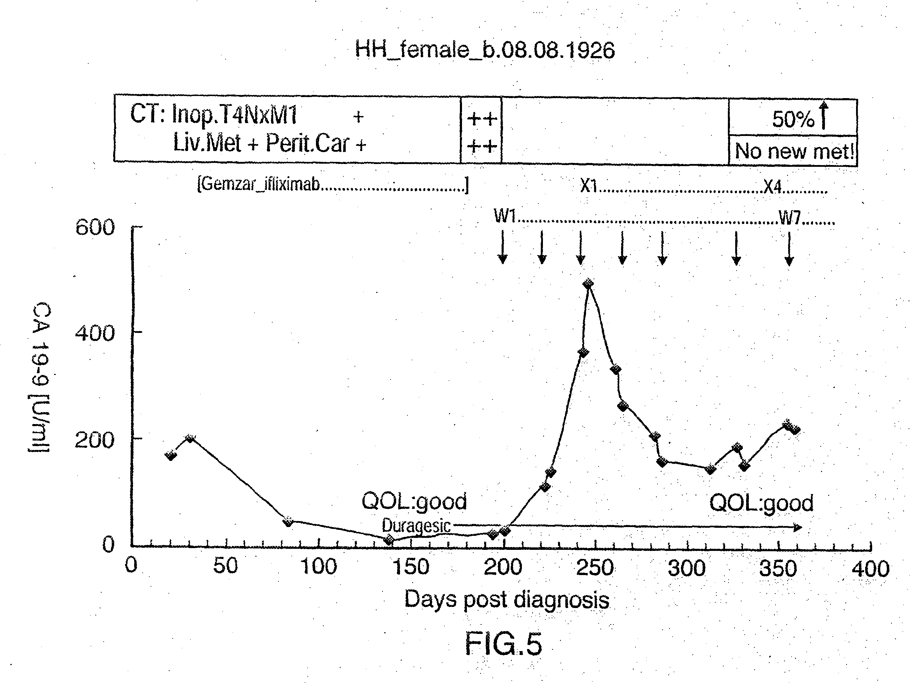

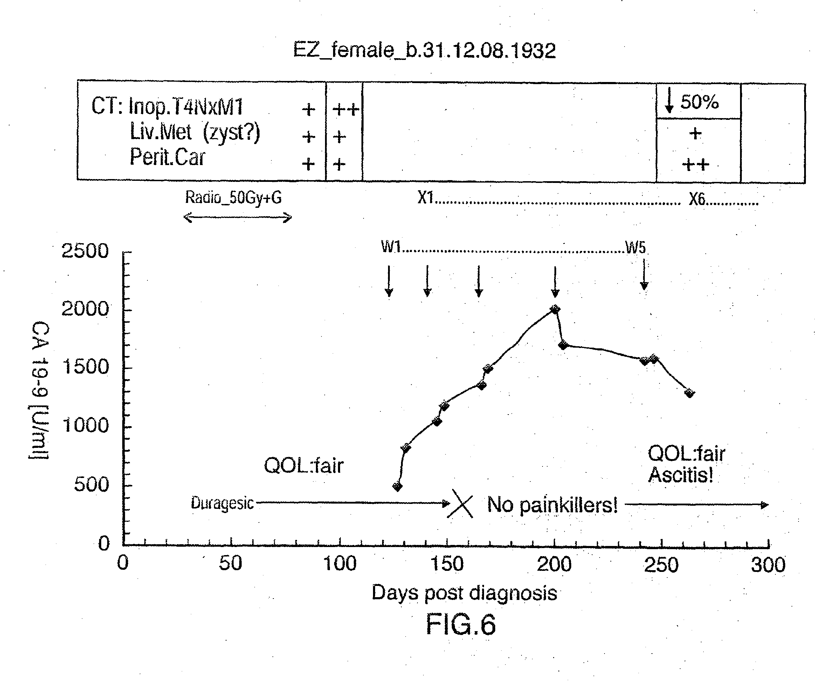

Figures 3-6 each illustrate the changes in levels of Carbohydrate Antigen 19-9 (CA 19-9), a marker for pancreatic cancer in patients treated with anti-cancer medications with and without WF-10, and also shows the changes in QOL (Quality of Life) during treatment.

### DETAILED DESCRIPTION OF THE PREFERRED EMBODIMENTS

The present inventors surprisingly have found that administration of a stabilized chlorite solution, such as WF10, to patients undergoing cancer chemotherapy with 5-FU or a 5-FU prodrug improves clinical outcomes and dramatically improves the patients Quality of Life index (QOL). The cancers that can be treated in this fashion includes cancers of the pancreas, gastrointestinal tract, head, neck, and breast.

5-FU and prodrugs such as capecitabine are described in United States patents 4,966,891 and 5,472,949, the contents of which are incorporated herein by reference in their entireties. Accordingly, in an embodiment of the invention, the prodrugs of 5-FU include a compound of the formula (I): 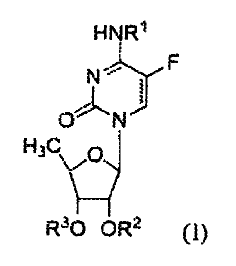 wherein R¹, R² and R³ independently represent a hydrogen, or an easily hydrolyzable radical under physiological conditions, with the proviso that, at least one of radicals represented by the symbol R¹, R² or R³ represents an easily hydrolyzable radical under physiological conditions; as well as hydrates or solvates of the compounds of the general formula (I). Preferably, the easily hydrolyzable radical is represented by the formula, R⁴CO- , R⁵OCO- or R⁶SCO- wherein R⁴ represents hydrogen atom, an alkyl, cycloalkyl, oxoalkyl, alkenyl, aralkyl or aryl radical, and R⁵ and R⁶ represent an alkyl or aralkyl radical, as well as hydrates and salts of the compounds of formula (I).

In a preferred embodiment of the invention, the prodrugs of 5-FU include a compound of the formula (II): 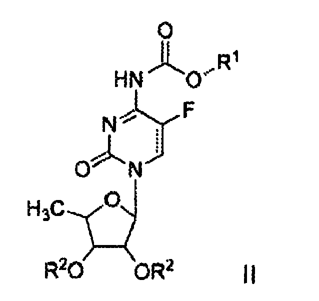 wherein R¹ is a radical of the formula -(CH₂)ₙ-Y, wherein Y is a cyclohexyl radical. a C₁-C₄alkoxy radical and, when Y is a cyclohexyl radical, n is an integer from 0 to 4 and when Y is a C₁-C₄alkoxy radical, n is an integer from 2 to 4, and R² is a hydrogen atom or C(O)O-R¹, as well as hydrates and salts of the compounds of formula (II).

In another preferred embodiment, the prodrugs of 5-FU are selected from the group consisting of:
N⁴-acetyl-5′-deoxy-5-fluorocytidine;
5′-deoxy-5-fluoro-N⁴ -propionylcytidine:
N⁴ -butyryl-5′-deoxy-5-fluorocytidine;
5′-deoxy-5-fluoro-N⁴ -isobutyrylcytidine:
5′-deoxy-5-fluoro-N⁴ -(2-methylbutyryl)cytidine;
5′-deoxy-N⁴ -(2-ethylbutyryl)-5-fluorocytidine:
5′-deoxy-N⁴ -(3,3-dimethylbutyryl)-5-fluorocytidine:
5′-deoxy-5-fluoro-N⁴ -pivaloylcytidine;
5′-deoxy-5-fluoro-N⁴ -valerylcytidine:
5′-deoxy-5-fluoro-N⁴ -isovalerylcytidine;
5′-deoxy-5-fluoro-N⁴ -(2-methylvaleryl)cytidine:
5′-deoxy-5-fluoro-N⁴ -(3-methylvaleryl)cytidine;
5′-deoxy-5-fluoro-N⁴ -(4-methylvaleryl)cytidine:
5′-deoxy-5-fluoro-N⁴-hexanoylcytidine;
5′-deoxy-5-fluoro-N⁴ -heptanoylcytidine;
5′-deoxy-5-fluoro-N⁴ -octanoylcytidine;
5′-deoxy-5-fluoro-N⁴ -nonanoylcytidine;
5′-deoxy-5-fluoro-N⁴ -hexadecanoylcytidine:
N⁴ -benzoyl-5′-deoxy-5-fluorocytidine;
5′-deoxy-5-fluoro-N⁴ -(4-methylbenzoyl)cytidine;
5′-deoxy-5-fluoro-N⁴ -(3-methylbenzoyl)cytidine:
5′-deoxy-5-fluoro-N⁴ -(2-methylbenzoyl)cytidine;
5′-deoxy-N⁴ -(4-ethylbenzoyl)-5-fluorocytidine:
5′-deoxy-N⁴ -(3,4-dimethylbenzoyl)-5-fluorocytidine;
5′-deoxy-N⁴-(3,5-dimethylbenzoyl)-5-fluorocytidine:
5′-deoxy-5-fluoro-N⁴ -(4-methoxybenzoyl)cytidine;
5′-deoxy-N⁴ -(3,4-dimethoxybenzoyl)-5-fluorocytidine:
5′-deoxy-N⁴ -(3,5-dimethoxybenzoyl)-5-fluorocytidine:
5'-deoxy-5-fluoro-N⁴-(3,4,5-trimethoxybenzoyl)cytidine:
5′-deoxy-5-fluoro-N⁴-(3,4,5-triethoxybenzoyl)cytidine:
5′-deoxy-N⁴ -(4-ethoxybenzoyl)-5-fluorocytidine;
5′-deoxy-5-fluoro-N⁴ -(4-propoxybenzoyl)cytidine;
5′-deoxy-N⁴-(3,5-diethoxybenzoyl)-5-fluorocytidine:
N⁴ -(4-chlorobenzonyl)-5′-deoxy-5-fluorocytidine:
5′-deoxy-N⁴-(3,4-dichlorobenzoyl)-5-fluorocytidine:
5′-deoxy-N⁴-(3,5-dichlorobenzoyl)-5-fluorocytidine:
5′-deoxy-5-fluoro-N⁴ -(4-nitrobenzoyl)cytidine;
5′-deoxy-5-fluoro-N⁴-(4-methoxycarbonylbenzoyl)cytidine:
N⁴-(4-acetylbenzoyl)-5′-deoxy-5-fluorocytidine:
5′-deoxy-5-fluoro-N⁴-(phenylacetyl)cytidine:
5′-deoxy-5-fluoro-N⁴-(4-methoxyphenylacetyl)cytidine:
5′-deoxy-5-fluoro-N⁴-nicotinoylcytidine;
5′-deoxy-5-fluoro-N⁴-isonicotinoylcytidine:
5′-deoxy-5-fluoro-N⁴-picolinoylcytidine:
5′-deoxy-5-fluoro-N⁴-(2-furoyl)cytidine:
5'-deoxy-5-fluoro-N⁴-(5-nitro-2-furoyl)cytidine:
5′-deoxy-5-fluoro-N⁴ -(2-thenoyl)cytidine;
5′-deoxy-5-fluoro-N⁴ -(5-methyl-2-thenoyl)cytidine;
5′-deoxy-5-fluoro-N⁴ -(1-methyl-2-pyrrolecarbonyl)cytidine:
5′-deoxy-5-fluoro-N⁴ -(3-indolylacetyl)cytidine:
N⁴ -(3-butenoyl)-5′-deoxy-5-fluorocytidine;
5′-O-benzoyl-5′-deoxy-5-fluorocytidine;
N⁴:3′-O-dibenzoyl-5′-deoxy-5-fluorocytidine;
5′-deoxy-N⁴ -(ethylthio)carbonyl-5-fluorocytidine;
5′-deoxy-5-fluoro-N⁴ -octadecanoylcytidine;
N⁴ -cyclopropanecarbonyl-5′-deoxy-5-fluorocytidine;
N⁴ -cyclohexanecarbonyl-5′-deoxy-5-fluorocytidine;
N⁴ -(1-adamantanecarbonyl)-5′-deoxy-5-fluorocytidine:
5′-deoxy-5-fluoro-N⁴ -(2-methoxybenzoyl)cytidine;
5′-deoxy-N⁴ -(2,4-dimethoxybenzoyl)-5-fluorocytidine:
5′-deoxy-5-fluoro-N⁴ -piperonyloylcytidine;
5′-deoxy-5-fluoro-N⁴ -(4-fluorobenzoyl)cytidine;
N⁴ -(2-chlorobenzoyl)-5′-deoxy-5-fluorocytidine;
N⁴ -(3-chlorobenzoyl)-5′-deoxy-5-fluorocytidine;
5′-deoxy-5-fluoro-N⁴ -(3-nitrobenzoyl)cytidine:
5′-deoxy-5-fluoro-N⁴ -[4-(methylthio)benzoyl]cytidine:
5′-deoxy-5-fluoro-N⁴ -(2-naphthoyl)cytidine:
5′-deoxy-5-fluoro-N⁴ -(3-furoyl)cytidine;
5′-deoxy-5-fluoro-N⁴ -(3-phenylpropionyl)cytidine:
N⁴ -cinnamoyl-5′-deoxy-5-fluorocytidine;
2', 3′-di-0-benzoyl-5′-deoxy-5-fluorocytidine:
N⁴,2′-O,3′-O-tribenzoyl-5′-deoxy-5-fluorocytidine:
5′-deoxy-5-fluoro-N⁴-(octyloxycarbonyl)cytidine:
N⁴ -(benzyloxycarbonyl)-5′-deoxy-5-fluorocytidine;
5′-deoxy-5-fluoro-N⁴ formylcytidine;
5′-deoxy-5-fluoro-N⁴-(propoxycarbonyl)cytidine:
N⁴ -(butoxycarbonyl)-5′-deoxy-5-fluorocytidine;
5′-deoxy-5-fluoro-N⁴ -(pentyloxycarbonyl)cytidine;
5′-deoxy-5-fluoro-N⁴ -(hexyloxycarbonyl)cytidine;
5′-deoxy-5-fluoro-N⁴ -(isopentyloxycarbonyl)cytidine;
5′-deoxy-5-fluoro-N⁴ -(neopentyloxycarbonyl)cytidine;
5′-deoxy-5-fluoro-N⁴-[(1,1,2-trimethylpropoxy)carbonyl]cytidine:
5′-deoxy-N⁴-[(3,3-dimethylbutoxy)carbonyl]-5-fluorocytidine;
5′-deoxy-5-fluoro-N⁴ -[(1-isopropyl-2-methylpropoxy)carbonyl]cytidine:
5′-deoxy-N⁴ -[(2-ethylbutoxy)carbonyl)-5-fluorocytidine;
N -[(cyclohexylmethoxy)carbonyl]-5′-deoxy-5-fluorocytidine;
5′-deoxy-5-fluoro-N⁴ -[(2-phenylethoxy)carbonyl]cytidine;
2′,3′-di-O-acetyl-5′-deoxy-5-fluoro-N⁴-(propoxycarbonyl)cytidine;
2′,3′-di-acetyl-N⁴ -(butoxycarbonyl)-5′-deoxy-5-fluorocytidine;
2′,3′-di-benzoyl-N⁴-(butoxycarbonyl)-5′-deoxy-5-fluorocytidine;
2′,3′-di-O-acetyl-5′-deoxy-5-fluoro-N⁴-(pentyloxycarbonyl)cytidine;
2′,3′-di-acetyl-5′-deoxy-5-fluoro-N⁴-(isopentyloxycarbonyl)cytidine;
2′,3′-di-O-acetyl-5′-deoxy-5-fluoro-N⁴ -(hexyloxycarbonyl)-cytidine;
2′,3′-di-O-acetyl-5′-deoxy-N⁴ -[(2-ethylbutyl)oxycarbonyl]-5 -fluorocytidine;
2′,3′-di-O-acetyl-N⁴-[(cyclohexylmethoxy)carbonyl]-5′-deoxy-5-fluorocytidine;
2′,3′-di-O-acetyl-5′-deoxy-5-fluoro-N⁴-[(2-phenylethoxy)carbonyl]cytidine:
5′-deoxy-5-fluoro-N⁴ -(isobutoxycarbonyl)cytidine;
5′-deoxy-5-fluoro-N⁴-[(2-propylpentyl)oxycarbonyl]cytidine;
5'-deoxy-N⁴ -[(2-ethylhexyl)oxycarbonyl]-5-fluorocytidine:
5′-deoxy-5-fluoro-N⁴-(heptyloxycarbonyl)cytidine;
N⁴ -[(2-cyclohexylethoxy)carbonyl]-5′-deoxy-5-fluorocytidine;
N⁴ -[(3-cyclohexylpropyl)oxycarbonyl]-5′-deoxy-5-fluorocytidine:
N⁴ -(cyclohexyloxycarbonyl)-5′-deoxy-5-fluorocytidine:
5′-deoxy-5-fluoro-N⁴-[(3-phenylpropyl)oxycarbonyl]cytidine: and
5′-deoxy-5-fluoro-N⁴ -[(2-methoxyethoxy)carbonyl]cytidine.
as well as hydrates and salts thereof.

In another preferred embodiment of the invention, the cytotoxic agent comprises a prodrug of 5-FU and is selected from the group consisting of Doxifluridine, UFT, S-1, BOF-A2 and mixtures thereof.

Other 5-FU prodrugs are known in the art and the skilled artisan will recognize that the methods described herein are applicable to any such prodrug, whether now known or discovered in the future. Although the methods described below relate to capecitabine, the skilled artisan will recognize that they apply equally to any 5-FU prodrug unless specifically indicated to the contrary.

Accordingly, in a preferred embodiment of the invention, the cytotoxic agent comprises a compound of the formula: 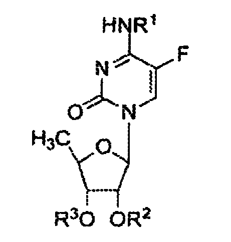 wherein R¹, R² and R³ are each independently hydrogen, or an easily hydrolyzable radical under physiological conditions, with the proviso that, at least one of R¹, R² or
R³ is an easily hydrolyzable radical under physiological conditions; as well as hydrates or solvates of the compounds of the general formula (I).

Preferably, the easily hydrolyzable radical is selected from the group consisting of

R⁴CO-, R⁵OCO- or R⁶SCO-

wherein R⁴ represents hydrogen, alkyl, cycloalkyl, oxoalkyl, alkenyl, aralkyl or aryl; R⁵ is alkyl or aralkyl radical; and R⁶ is alkyl or aralkyl.

As throughout this specification, the term "alkyl" refers to straight or branched chain having 1 to 19 carbon atoms and is preferably selected from the group consisting of methyl, ethyl, propyl, isopropyl, butyl, isobutyl, sec-butyl, tert-butyl, pentyl, isopentyl, neopentyl, hexyl, isohexyl, heptyl, octyl, nonyl, decyl, undecyl, dodecyl, tridecyl, tetradecyl, pentadecyl, hexadecyl, heptadecyl and nonadecyl.

Further, as used throughout this specification, the term "cycloalkyl" is a member selected from the group consisting of cyclopropyl, cyclobutyl, cyclopentyl, cyclohexyl and adamantyl.

Still further, as used throughout this specification, the term "oxoalkyl" is a member selected from the group consisting of acetyl, propionyl, butyryl, 2-oxopropyl, 2-oxobutyl and 3-oxobutyl.

Still further, as used throughout this specification, the term "alkenyl" means an unsubstituted or substituted alkenyl radical having 3 to 19 carbon atoms and is preferably selected from the group consiting of allyl, butenyl, 3-methyl-2-butenyl, 1-methyl-2-propenyl, hexenyl, decenyl, undecenyl, tridecenyl, pentadecenyl, heptadecenyl, heptadecadienyl, heptadecatrienyl, nonadecenyl, nonadecadienyl, nonadecatetraenyl and 2-phenylvinyl.

Still further, as used throughout this specification, the term "aralkyl" means an unsubstituted or substituted aralkyl radical selected from the group consisting of benzyl, 1-phenylethyl, methylbenzyl, tluorobenzyl, chlorobenzyl, methoxybenzyl, dimethoxybenzyl, nitrobenzyl, phenethyl, picolyl and 3-indolylmethyl.

Still further, as used throughout this specification, the term "aryl" means an unsubstituted or substituted aryl radical such as phenyl, tolyl, xylyl, mesityl, cumenyl, ethylphenyl, fluorophenyl, chlorophenyl, bromophenyl, iodophenyl, difluorophenyl, dichlorophenyl, methoxyphenyl, dimethoxyphenyl, trimethoxyphenyl, ethoxyphenyl, diethoxyphenyl, triethoxyphenyl, propoxyphenyl, methylenedioxyphenyl, (methylthio)phenyl, nitrophenyl, cyanophenyl, acetylphenyl, carbamoylphenyl, methoxycarbonylphenyl, naphthyl, biphenylyl, thienyl, methylthienyl, furyl, nitrofuryl, pyrrolyl, methylpyrrolyl, imidazolyl, pyrazoly-1, pyridyl, methylpyridyl and pyrazinyl.

The most preferred cytotoxic agent for use in the present invention is capecitabine.

Capecitabine for monotherapy generally is administered orally at a dose of about 2500 mg/m²/day, divided into two equal doses, for 2 weeks, followed by a 1-week rest period. The product is supplied commercially in 150 mg and 500 mg tablets. The tablets are administered at the rate of about 1 to about 4 times a day during the treatment period. The daily doses of capecitabine will range from about 1000 mg/ m² to about 3500 mg/ m² per day in the combinations of this invention.

WF10 may be administered at any time before, during, or after the treatment with capecitabine. For example, WF10 administration can begin from 1-4 weeks prior to the beginning of the capecitabine treatments, can begin simultaneously with the capecitabine treatments, or can be commenced or continued for up to 14 days after capecitabine treatment is ended.

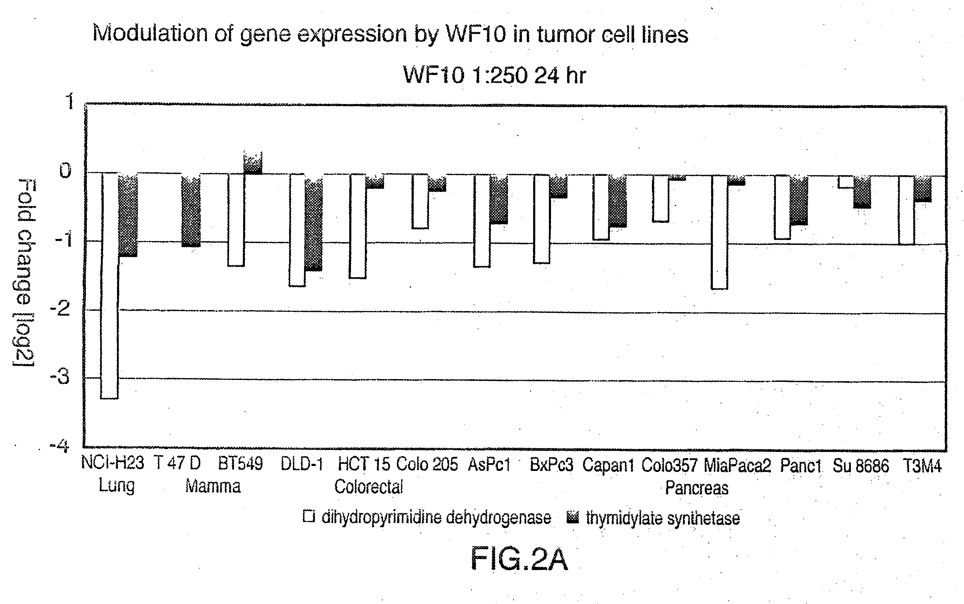

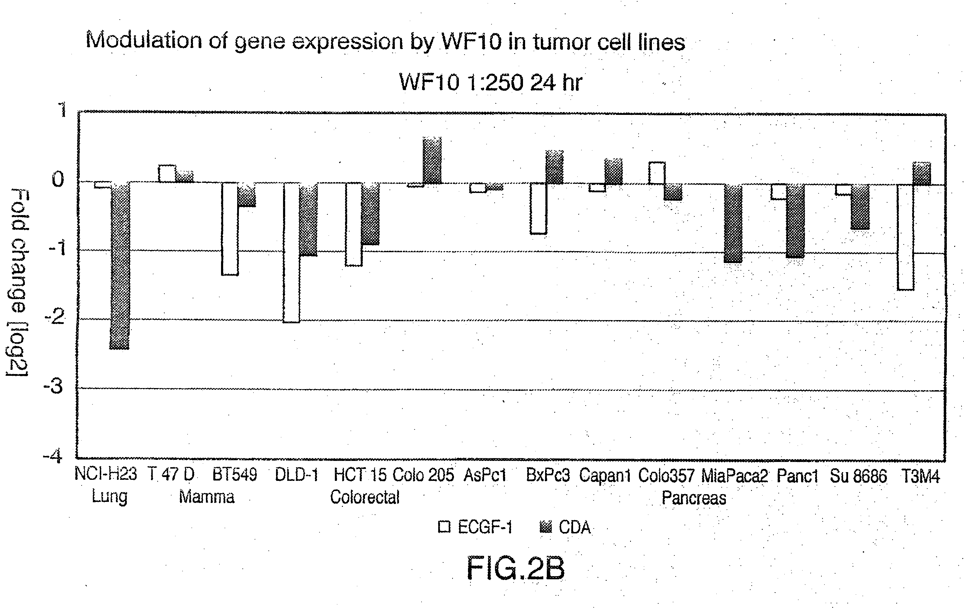

Administration of WF10 surprisingly has been found to not only improve a patient's QOL, but also appears to improve clinical outcomes in the patients. The EORTC QLQ-C30 Quality of Life Questionnaire is available at: http://www.eortc.be/home/qol/ExplQLQ-C30.htm and may be used in evaluating a patient's QOL. Without being bound by any theory, the present inventors believe that this enhanced efficacy is due to WF10′s ability to inhibit one or more of the enzymes involved in the degradation of 5-FU *in vivo*. In a first set of experiments the influence of WF10 on the gene expression of cytidine deaminase (CyD), thymidine phosphorylase (ECGF-1), ihymidylate synthase and dihydropyrimidine dehydrogenase (DPD) was studied in 14 cell lines including 8 lines of pancreatic origin in vitro (see Figure 2.) DPD is a key enzyme in the breakdown and inactivation of 5FU to FdUMP.

Capecitabine does not incorporate a DPD inhibitor. More than 80% of an administered dose of 5-FU is eliminated by catabolism through DPD which is the rate-limiting enzyme of pyrimidine catabolism. Surprisingly, it was found that DPD expression was inhibited by WF10 in multiple tumor cell lines studied. The combination of DPD inhibitors with 5-FU itself or a 5-FU prodrug has several potential pharmacologic benefits conferred by the inhibition of 5-FU catabolism during gastrointestinal absorption and first pass in the liver. Furthermore, DPD inhibition improves pharmacokinetic behaviour of delivered 5-FU by reducing interpatient variability and by increasing 5-FU half-life. This latter benefit is particularly useful in reducing the total dosage of 5-FU or its prodrug thereby limiting the systemic toxicity of 5-FU and avoiding the inconvenience of extended or frequent infusions of 5-FU or repeated oral administrations of an oral prodrug.

### Stabilized chlorite solutions suitable for use in conjunction with 5-FU or 5-FU prodrugs

A preferred embodiment of the treatment of this invention entails administration to a mammal in need thereof of WF10, an aqueous stabilized chlorite solution. WF10 is a 10% aqueous dilute solution of OXO-K993, which is analytically characterized as a solution containing the ions chlorite (4.25%), chloride (2.0%), chlorate (1.5%), sulfate (0.7%), and sodium (4.0%). 1 g of OXO-K993 contains 42.5 mg (630 µmol) of the active chlorite ion. I mL of WF10 solution contains 4.25 mg (63 µmol) of the active chlorite ion. At room temperature, OXO-K-993 is a colourless or slightly yellow, clear, aqueous solution. WF10 is available in multi-dose glass vials for intravenous infusion from Dimethaid Gmbh in Wanzleben Germany. In the original sealed bottle WF10 is stable for up to 60 months.

The preparation of OXO-K993 first requires mixture of sodium chlorite (NaClO₂) and sodium hypochlorite (NaOCl) in a molar ratio of 4.8 to 1 in Water for Injection (WFI). The pH of the solution should be greater than pH 11.0. After addition of the catalyst chlorylsulfuric acid, the following reactions occurs:

2 ClO₂ + OCl + 2 H⁺ → 2 ClO₂ + Cl⁻ + H₂O (1)

The pH of the solution decreases to between 6.5 and 7.1. A portion of the chlorite is oxidized to chlorine dioxide (ClO₂) in the redox process described by Equation (1). In an equilibrium reaction, the developing chlorine dioxide forms an intense brown charge-transfer complex with the excess unoxidized chlorite, as shown in Equation (2):

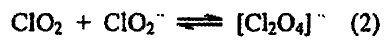

The maximum yield is obtained after a reaction time of approximately 60 to 90 minutes. After this time, the charge-transfer complex begins to decompose.

In the next step of the synthesis, 9.65 mmol (per kg of the reaction solution) of sodium carbonate peroxohydrate (2 Na₂CO₃•3 H₂O₂) is added to the solution, at which time the color of the solution becomes a brighter yellow. Upon addition of sodium carbonate peroxohydrate, part of the chlorine dioxide is reduced back to chlorite, and oxygen is formed simultaneously:

2 ClO₂ + H₂O₂ + 2 OH⁻ → 2 ClO₂⁻ + O₂ + 2 H₂O (3)

After another 15 minutes, 102 mmol (per kg of the reaction solution) of sodium peroxide (Na₂O₂) is added to the solution, which becomes completely decolorized as the remaining chlorine dioxide is reduced completely to chlorite. From sodium peroxide, oxygen evolves in a slow and continuous process:

2 O₂²⁻ + 2 H₂O → O₂ + 4 OH⁻ (4)

The evolution of oxygen typically requires at least 4 weeks. Simultaneously, hydroxyl ions are formed, resulting in a high pH value (pH > 13) of the solution, which thereby stabilizes the active substance chlorite.

The final reaction product, OXO-K993, resulting from this synthesis is a stable aqueous solution, which contains the active substance, chlorite (4,25%), together with the anions chloride (2.0%), chlorate (1.5%), and sulfate (0.7%) and sodium as the cation.

As used throughout this specification, the term "stabilized chorite solution" is intended to encompass a solution which, after being stored in the dark at a temperature of less than 30°C for a period of at least 6 months after production of the solution, contains chlorite in an amount of at least about 70%, preferably at least about 80%, more preferably at least about 90% even more preferably at least about 95%, most preferably at least about 98%, based on the amount of chlorite present immediately after production of the solution. More preferably, the solution has these amounts of chlorite at least 36 months after production of the solution, most preferably at least 60 months after production of the solution.

The skilled artisan will recognize that any chemically stabilized chlorite solution can be used in the various embodiments of the present invention, and that the scope of the invention is not limited to use of the specific product described above.

The dosage of the stabilized chlorite preparation that is administered to a patient to achieve a desired therapeutic result will depend upon various factors, including the body weight and gender of the patient. Methods of adjusting dosage regimens to take body weight, gender, and other metabolic factors into account are well known in the art. The particular therapeutic endpoint that is to be achieved will vary depending upon the particular pathology and symptoms of the disease that is being treated, but these endpoints are well known in the art. For example, in treating a patient with cancer one would evaluate tumor response, patient survival and side effects of co-therapy with another primary anti-cancer drug. Pancreatic cancer is associated with elevated levels of CA19-9 antigen. Efficacy of treatment using the combination of WF10 and a 5-FU drug or prodrug such as capecitabine or the like may be estimated by measuring levels of CA19-9 both before and after treatment. Similarly, patients suffering from prostate cancer display a high titer of prostate specific antigen (PSA), and a reduction in this titer following treatment is one indication of the efficacy of the treatment. The skilled artisan readily will appreciate, however, that clinical benefit often may readily be ascertained by observing general improvement in the symptoms reported by a patient, without the need for a quantitative measurement of clinical response. Similarly, absence of a measurable response in certain laboratory findings does not of itself preclude the existence of clinically significant benefit.

In the context of the present invention, those skilled in the art will appreciate that the term "an effective amount" as applied to the stabilized chlorite solution indicates an amount of solution which, when administered in vivo to a subject, will bring about a clinically relevant change in a clinical marker of a disease or will provide a measurable change in the subject's QOL. Typically, an inhibition effective amount of the chlorite solution will vary between about 0.1 ml/kg to about 1.5 ml/kg, preferably, about 0.5 ml/kg of body weight and at a concentration of about 40 to about 80 mMol ClO₂⁻ per liter, preferably about 60 mMol ClO₂⁻ per liter, respectively.]. The skilled artisan will recognize however, that higher or lower doses may be effective in certain subjects or in certain circumstances.

Preferably, the chlorite solution of the invention is administered once daily for anywhere from about three to seven days, preferably five days, followed by a period of rest of from 10 to 20 days, preferably from 14-18 days, and more preferably, 16 days, to constitute one cycle during the initial phase of treatment. Preferably, patients are treated with more than one cycle, more preferably, at least three cycles, and most preferably, at least five cycles. The skilled artisan will recognize, however, that other regimens are possible, and may in fact be preferable. Methods of manipulating such regimens are well known in the art.

For example, an alternative treatment regimen consists of intravenously administering the stabilized chlorite solution of the invention once daily for a period of five days, followed by two days of rest (e.g., over the weekend), followed by five more consecutive days of administration, followed by a period of rest from anywhere between 1 and 4 weeks to constitute one cycle. Preferably, patients are treated with more than one cycle, more preferably more than three. As the patient responds to a course of multiple cycles of chlorite solution, the rest period may be extended beyond 4 weeks. Skilled artisans are capable of modifying the administration of the stabilized chlorite solution of the invention depending on the disease treated and the size of the patient, using the guidelines provided herein.

The present invention, thus generally described, will be understood more/readily by reference to the following examples, which are provided by way of illustration and are not intended to be limiting of the present invention.

### EXAMPLE 1: CASE REPORT #1 - IS

A 63-year-old woman was diagnosed with pancreatic cancer (inoperable; T4N0M0) and initially received a course of combined radiation and chemotherapy (50Gy+Gemzar+Cisplatin), which failed to stop tumor progression and yielded a very poor quality of life (QOL). Initiation of WF10-Xeloda treatment (at 3 months post diagnosis) led to an impressive improvement in QOL and a positive objective response as measured by reduction of the primary tumor mass by 50%, decompression of blood vessels and drop of the tumor marker (CA19-9) to below the upper limit of normal (<37 U/ml). After 9 months, a rise of CA19-9, an increase of tumor size as well as CT-detected solitary liver lesion required the change of chemotherapy from Xeloda to Gemzar and the additional management of the pain syndrome by Duragesic. To date, the patient is alive (over 15 months post diagnosis), still receiving WF10-Gemzar therapy (12 months in WF10-linked therapy), robust and having good QOL.

### EXAMPLE 2: CASE REPORT #2 HH

A 78-year-old woman was diagnosed with pancreatic cancer (OP: inoperable, advanced liver and peritoneal metastasis; T4M1Nx) and initially received a course of Gemzar+Infliximab. After initial stabilization, rapid tumor progression, vessel obstruction and declining QOL were observed. WF10-Xeloda treatment was started at 6 months post diagnosis and resulted in the improvement of QOL, stabilization of serum CA19-9 level and absence of new liver lesions. Slow progression of the primary tumor lesion was detected by CT-scan 5 months later. Pain control stayed at a constant level. Due to development of Xeloda-related side effects, progressive dose-reduction was instituted and eventually complete cessation of Xeloda was required. Application of WF10 as a single agent allowed the patient to stay in stable condition for the next 2 months, but then general conditions deteriorated. Therapy was terminated and the patient died a few weeks later. Summary: survival time of 14 months post diagnosis (8 months in WF10-linked therapy); good QOL for a long period of time despite tumor progression.

### EXAMPLE 3: CASE REPORT #3 - EZ

A month after initial diagnosis of pancreatic cancer (inoperable, advanced liver and peritoneal metastasis; T4NxM1), a 72-year-old woman underwent combined radiation and chemotherapy (50 Gy+ Gemzar + Gemzar post radio). After 4 months, disease progression yielded rapidly progressing metastasis, declining QOL and the need to institute pain control with narcotics. Initiation of WF10-Xeloda treatment resulted in improved QOL, discontinuation of Duragesic use, stabilization of CA19-9 level, reduction of the primary tumor volume by half and the absence of new liver metastases. After 6 months, however, progressing peritoneal metastasis led to ascites development. The patient had a number of tumor-unrelated diseases, which became increasingly difficult to control and finally led to hospitalization and death. Summary: survival time of 12 months post diagnosis (6.5 months in WF10-linked therapy); improved QOL for a long period of time despite metastasis and numerous additional health problems; complete withdrawal of painkillers; reduction of the primary tumor.

### EXAMPLE 4: CASE REPORT #4 - JCJ

A 51-year-old man was diagnosed with pancreatic cancer, underwent tumor resection (T4N1Mx) and received 5FU + Leucovorin as adjuvant therapy. He underwent another operation 6 months later due to GI complications. Because of extremely poor QOL and increasing serum CA19-9 level, the therapy regimen was changed to WF10+Gemzar. As a result, immediate improvement of QOL and decline of CA19-9 under the level of detection were observed. The patient stayed disease-free for 10 months. Thereafter, because of the emergence of peritoneal and liver metastases, the therapy regimen was changed to WF10-Xeloda, which allowed the patient to stay robust, maintain his weight, and have good QOL and working habits for another 6 months. Summary: Survival time of 22 months post diagnosis/OP (16 months post therapy initiation); very good QOL, robust conditions and maintenance of employment status.

While this invention has been described with reference to illustrative embodiments and examples, the description is not intended to be construed in a limiting sense. Thus, various modifications of the illustrative embodiments, as well as other embodiments of the invention, will be apparent to persons skilled in the art upon reference to this description. It is therefore contemplated that the appended claims will cover any such modifications or embodiments.

All publications, patents and patent applications referred to herein are incorporated by reference in their entirety to the same extent as if each individual publication, patent or patent application was specifically and individually indicated to be incorporated by reference in its entirety.

## Claims

1. An effective amount of a cytotoxic agent comprising a prodrug of 5-fluorouracil (5-FU) and a stabilized chlorite solution for use in the treatment of a neoplastic disorder.

2. The effective amount of a a cytotoxic agent according to claim 1. wherein the neoplastic disorder is a tumor of the gastrointestinal tract, head, neck, breast or pancreas.

3. The effective amount of a cytotoxic agent according to claim 1 or 2, wherein the stabilized chlorite solution comprises WF10.

4. The effective amount of a cytotoxic agent according to any one of claims 1 to 3.
wherein the cytotoxic agent comprising a prodrug of 5-fluorouracil (5-FU) is a compound of the formula II: 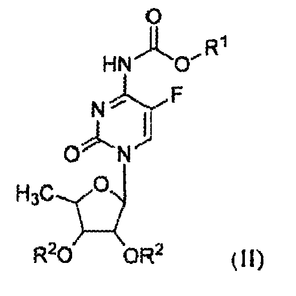 wherein R¹ is a radical of the formula -(CH₂)ₙ-Y, wherein Y is a cyclohexyl radical, a C₁-C₄alkoxy radical and, when Y is a cyclohexyl radical, n is an integer from 0 to 4 and when Y is a C₁-C₄alkoxy radical. n is an integer from 2 to 4, and R² is a hydrogen atom or C(O)O-R¹, and mixtures thereof.

5. The effective amount of a cytotoxic agent according to any one of claims 1 to 3, wherein the cytotoxic agent comprising a prodrug of 5-FU is selected from the group consisting of Doxifluridine, UFT, S-1, BOF-A2, and mixtures thereof, as well as:
N⁴ -acetyl-5′-deoxy-5-fluorocytidine;
5′-deoxy-5-fluoro-N⁴ propionylcytidine;
N⁴ -butyryl-5′-deoxy-5-fluorocytidine;
5′-deoxy-5-fluoro-N⁴ -isobutyrylcytidine;
5′-deoxy-5-fluoro-N⁴ -(2-methylbutyryl)cytidine;
5′-deoxy-N⁴ -(2-ethylbutyryl)-5-fluorocytidine;
5′-deoxy-N⁴ -(3,3-dimethylbutyryl)-5-fluorocytidine;
5′-deoxy-5-fluoro-N⁴ -pivaloylcytidine;
5′-deoxy-5-fluoro-N⁴ -valerylcytidine;
5′-deoxy-5-fluoro-N⁴ -isovalerylcytidine;
5′-deoxy-5-fluoro-N⁴ -(2-methylvaleryl)cytidine;
5′-deoxy-5-fluoro-N⁴ -(3-methylvaleryl)cytidine;
5′-deoxy-5-fluoro-N⁴ -(4-methylvaleryl)cytidine;
5′-deoxy-5-fluoro-N⁴ -hexanoylcytidine;
5′-deoxy-5-fluoro-N⁴ -heptanoylcytidine;
5′-deoxy-5-fluoro-N⁴ -octanoylcytidine;
5′-deoxy-5-fluoro-N⁴ -nonanoylcytidine;
5′-deoxy-5-fluoro-N⁵ -hexadecanoylcytidine;
N⁴-benzoyl-5′-deoxy-5-fluorocytidine;
5′-deoxy-5-fluoro-N⁴ -(4-methylbenzoyl)cytidine;
5′-deoxy-5-fluoro-N⁴ -(3-methylbenzoyl)cytidine;
5′-deoxy-5-fluoro-N⁴ -(2-methylbenzoyl)cytidine;
5′-deoxy-N⁴ -(4-ethylbenzoyl)-5-fluorocytidine;
5′-deoxy-N⁴ -(3,4-dimethylbenzoyl)-5-fluorocytidine;
5′-deoxy-N⁴ -(3,5-dimethylbenzoyl)-5-fluorocytidine;
5′-deoxy-5-fluoro-N⁴ -(4-methoxybenzoyl)cytidine;
5′-deoxy-N⁴ -(3,4-dimethoxybenzoyl)-5-fluorocytidine;
5′-deoxy-N⁴ -(3,5-dimethoxybenzoyl)-5-fluorocytidine;
5′-deoxy-5-fluoro-N⁴-(3,4,5-trimethoxybenzoyl)cytidine;
5′-deoxy-5-fluoro-N⁴ -(3,4.5-triethoxybenzoyl)cytidine;
5′-deoxy-N⁴ -(4-ethoxybenzoyl)-5-fluorocytidine;
5′-deoxy-5-fluoro-N⁴ -(4-propoxybenzoyl)cytidine;
5′-deoxy-N⁴ -(3,5-diethoxybenzoyl)-5-fluorocytidine;
N⁴ -(4-chlorobenzoyl)-5′-deoxy-5-fluorocytidine;
5′-deoxy-N⁴ -(3,4-dichlorobenzoyl)-5-fluorocytidine;
5′-deoxy-N⁴ -(3,5-dichlorobenzoyl)-5-fluorocytidine;
5′-deoxy-5-fluoro-N⁴ -(4-nitrobenzoyl)cytidine;
5′-deoxy-5-fluoro-N⁴ -(4-methoxycarbonylbenzoyl)cytidine;
N⁴ -(4-acetylbenzoyl)-5′-deoxy-5-fluorocytidine;
5′-deoxy-5-fluoro-N⁴ -(phenylacetyl)cytidine;
5′-deoxy-5-fluoro-N⁴ -(4-methoxyphenylacetyl)cytidine;
5′-deoxy-5-fluoro-N⁴ -nicotinoylcytidine;
5′-deoxy-5-fluoro-N⁴ -isonicotinoylcytidine;
5′-deoxy-5-fluoro-N⁴ -picolinoylcytidine;
5′-deoxy-5-fluoro-N⁴ -(2-furoyl)cytidine;
5′-deoxy-5-fluoro-N⁴ -(5-nitro-2-furoyl)cytidine;
5′-deoxy-5-fluoro-N⁴ -(2-thenoyl)cytidine;
5′-deoxy-5-fluoro-N⁴ -(5-methyl-2-thenoyl)cytidine;
5′-deoxy-5-fiuoro-N⁴ -(1-methyl-2-pyrrolecarbonyl)cytidine;
5′-deoxy-5-fluoro-N⁴ -(3-indolylacetyl)cytidine;
N⁴ -(3-butenoyl)-5′-deoxy-5-fluorocytidine;
5′-O-benzoyl-5′-deoxy-5-fluorocytidine;
N⁴;3′-O-dibenzoyl-5′-deoxy-5-fluorocytidine;
5′-deoxy-N⁴ -(ethylthio)carbonyl-5-fluorocytidine;
5′-deoxy-5-fluoro-N⁴ -octadecanoylcytidine;
N⁴ -cyclopropanecarbonyl-5′-deoxy-5-fluorocytidine;
N⁴ -cyclohexanecarbonyl-5′-deoxy-5-fluorocytidine;
N⁴ -(1-adamantanecarbonyl)-5′-deoxy-5-fluorocytidine;
5′-deoxy-5-fluoro-N⁴ -(2-methoxybenzoyl)cytidine;
5′-deoxy-N⁴ -(2,4-dimethoxybenzoyl)-5-fluorocytidine;
5′-deoxy-5-fluoro-N⁴ -piperonyloylcytidine;
5′-deoxy-5-fluoro-N⁴ -(4-fluorobenzoyl)cytidine;
N⁴ -(2-chlorobenzoyl)-5′-deoxy-5-fluorocytidine;
N⁴ -(3-chlorobenzoyl)-5′-deoxy-5-fluorocytidine;
5′-deoxy-5-fluoro-N⁴ -(3-nitrobenzoyl)cytidine;
5′-deoxy-5-fluoro-N⁴ -[4-(methylthio)benzoyl]cytidine;
5′-deoxy-5-fluoro-N⁴ -(2-naphthoyl)cytidine;
5′-deoxy-5-fluoro-N⁴ -(3-furoyl)cytidine;
5′-deoxy-5-fluoro-N⁴ -(3-phenylpropionyl)cytidine;
N⁴ -cinnamoyl-5′-deoxy-5-fluorocytidine;
2′, 3'-di-0-benzoyl-5′-deoxy-5-fluorocytidine;
N⁴,2′-O,3′-O-tribenzoyl-5′-deoxy-5-fluorocytidine;
5′-deoxy-5-fluoro-N⁴ -(octyloxycarbonyl)cytidine;
N⁴ -(benzyloxycarbonyl)-5′-deoxy-5-fluorocytidine;
5′-deoxy-5-fluoro-N⁴ - formylcytidine;
5′-deoxy-5-fluoro-N⁴ -(propoxycarbonyl)cytidine;
N⁴ -(butoxycarbonyl)-5′-deoxy-5-fluorocytidine;
5′-deoxy-5-fluoro-N⁴ -(pentyloxycarbonyl)cytidine;
5′-deoxy-5-fluoro-N⁴ -(hexyloxycarbonyl)cytidine;
5′-deoxy-5-fluoro-N⁴ -(isopentyloxycarbonyl)cytidine;
5′-deoxy-5-fluoro-N⁴ -(neopentyloxycarbonyl)cytidine;
5′-deoxy-5-fluoro-N⁴ -[(1.1,2-trimethylpropoxy)carbonyl]cytidine;
5′-deoxy-N⁴ -[(3,3-dimethylbutoxy)carbonyl]-5-fluorocytidine;
5′-deoxy-5-fluoro-N⁴ -[(1-isopropyl-2-methylpropoxy)carbonyl]cytidine;
5′-deoxy-N⁴ -[(2-ethylbutoxy)carbonyl]-5-fluorocytidine;
N -[(cyclohexylmethoxy)carbonyl]-5′-deoxy-5-fluorocytidine;
5′-deoxy-5-fluoro-N⁴ -[(2-phenylethoxy)carbonyl]cytidine;
2′,3′-di-O-acetyl-5′-deoxy-5-fluoro-N⁴ -(propoxycarbonyl)cytidine;
2′,3′-di-acetyl-N⁴ -(butoxycarbonl)-5′-deoxy-5-fluorocytidine;
2′,3′-di-benzoyl-N⁴ -(butoxycarbonyl)-5′-deoxy-5-fluorocytidine;
2′,3′-di-O-acetyl-5′-deoxy-5-fluoro-N⁴ -(pentyloxycarbonyl)cytidine;
2′,3′-di-acetyl-5′-deoxy-5-fluoro-N⁴-(isopentyloxycarbonyl)cytidine;
2′,3′-di-O-acetyl-5′-deoxy-5-fluoro-N⁴ -(hexyloxycarbonyl)-cytidine;
2′,3′-di-O-acetyl-5′-deoxy-N⁴ -[(2-ethylbutyl)oxycarbonyl]-5 -fluorocytidine;
2′,3′-di-O-acetyl-N⁴ -[(cyclohexylmethoxy)carbonyl]-5′-deoxy-5-fluorocytidine;
2′,3′-di-O-acetyl-5′-deoxy-5-fluoro-N⁴ -[(2-phenylethoxy)carbonyl]cytidine;
5′-deoxy-5-fluoro-N⁴ -(isabutoxycarbanyl)cytidine;
5′-deoxy-5-fluoro-N⁴ -[(2-propylpentyl)oxycarbonyl]cytidine;
5′-deoxy-N⁴ -[(2-ethylhexyl)oxycarbonyl]-5-fluorocytidine;
5′-deoxy-5-fluoro-N⁴ -(heptyloxycarbonyl)cytidine;
N⁴ -[(2-cyclohexylethoxy)carbonyl]-5′-deoxy-5-fluorocytidine;
N⁴ -[(3-cyclohexylpropyl)oxycarbonil]-5′-deoxy-5-fluorocytidine;
N⁴ -(cyclohexyloxycarbonyl)-5′-deoxy-5-fluorocytidine;
5′-deoxy-5-fluoro-N⁴ -[(3-phenylpropyl)oxycarbonyl]cytidine;
5′-deoxy-5-fluoro-N⁴ -[(2-methoxyethoxy)carbonyl]cytidine, and
hydrates and salts thereof.

6. An effective amount of a stabilized chlorite solution for use in enhancing the efficacy of a cytotoxic agent in the treatment of a neoplastic disorder, the cytotoxic agent comprising a prodrug of 5-fluorouracil (5-FU), wherein the cytotoxic agent is degraded or inactivated by an enzyme in a subject, and the stabilized chlorite solution inhibits the expression or activity of the enzyme, preferably, dihydropyrimidine dehydrogenase.

7. The effective amount of a stabilized chlorite solution according to claim 6, wherein the neoplastic disorder is a tumor of the gastrointestinal tract, head, neck, breast or pancreas.

8. The effective amount of a stabilized chlorite solution according to claim 6 or 7, wherein the stabilized chlorite solution comprises WF10.

9. The effective amount of a stabilised chlorite solution according to any one of claims 6 to 8. wherein the cytotoxic agent comprising a prodrug of 5-fluorouracil (5-FU) is a compound of the formula II: 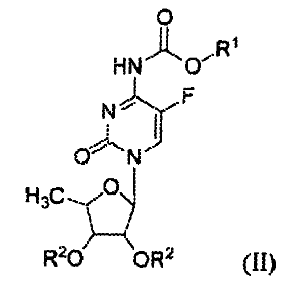 wherein R¹ is a radical of the formula -(CH₂)ₙ-Y, wherein Y is a cyclohexyl radical, a C₁-C₄alkoxy radical and, when Y is a cyclohexyl radical, n is an integer from 0 to 4 and when Y is a C₁-C₄alkoxy radical, n is an integer from 2 to 4, and R² is a hydrogen atom or C(O)O-R¹, and mixtures thereof.

10. The effective amount of a stabilized chlorite solution according to any one of claims 6 to 8, wherein the cytotoxic agent comprising a prodrug of 5-FU is selected from the group consisting of Doxifluridine, UFT, S-1, BOF-A2, and mixtures thereof, as well as:
N⁴ -acetyl-5′-deoxy-5-fluorocytidine;
5′-deoxy-5-fluoro-N⁴ -propionylcytidine;
N⁴ -butyryl-5′-deoxy-5-fluorocytidine;
5′-deoxy-5-fluoro-N⁴ -isobutyrylcytidine;
5′-deoxy-5-fluoro-N⁴ -(2-methylbutyryl)cytidine;
5′-deoxy-N⁴ -(2-ethylbutyryl)-5-fluorocytidine;
5′-deoxy-N⁴ -(3,3-dimethylbutyryl)-5-fluorocytidine;
5′-deoxy-5-fluoro-N⁴ -pivaloylcytiditie;
5′-deoxy-5-fluoro-N⁴ -valerylcytidine;
5′-deoxy-5-fluoro-N⁴ -isovalerylcytidine;
5′-deoxy-5-fluoro-N⁴ -(2-methylvaleryl)cytidine;
5′-deoxy-5-fluoro-N⁴ -(3-methylvaleryl)cytidine;
5′-deoxy-5-fluoro-N⁴ -(4-methylvaleryl)cytidine;
5′-deoxy-5-fluoro-N⁴ -hexanoylcytidine;
5′-deoxy-5-fluoro-N⁴ -heptanoylcytidine;
5′-deoxy-5-fluoro-N⁴ -octanoylcytidine;
5′-deoxy-5-fluoro-N⁴ -nonanoylcytidine;
5′-deoxy-5-fluoro-N⁴ -hexadecanoylcytidine;
N⁴ -benzoyl-5′-deoxy-5-fluorocytidine;
5′-deoxy-5-fluoro-N⁴ -(4-methylbenzoyl)cytidine;
5′-deoxy-5-fluoro-N⁴ -(3-methylbenzoyl)cytidine;
5′-deoxy-5-fluoro-N⁴ -(2-methylbenzoyl)cytidine;
5′-deoxy-N⁴ -(4-ethylbenzoyl)-5-fluorocytidine;
5′-deoxy-N⁴ -(3,4-dimethylbenzoyl)-5-fluorocytidine;
5′-deoxy-N⁴ -(3,5-dimethylbenzoyl)-5-fluorocytidine;
5′-deoxy-5-fluoro-N⁴ -(4-methoxybenzoyl)cytidine;
5′-deoxy-N⁴ -(3,4-dimethoxybenzoyl)-5-fluorocytidine;
5′-deoxy-N⁴ -(3,5-dimethoxybenzoyl)-5-fluorocytidine;
5′-deoxy-5-fluoro-N⁴ -(3,4,5-trimethoxybenzoyl)cytidine;
5′-deoxy-5-fluoro-N⁴ -(3,4,5-triethoxybenzoyl)cytidine;
5′-deoxy-N⁴ -(4-ethoxybenzoyl)-5-fluorocytidine;
5′-deoxy-5-fluoro-N⁴ -(4-propoxybenzoyl)cytidine;
5′-deoxy-N⁴ -(3,5-diethoxybenzoyl)-5-fluorocytidine;
N⁴ -(4-chlorobenzoyl)-5′-deoxy-5-fluorocytidine;
5′-deoxy-N⁴ -(3,4-dichlorobenzoyl)-5-fluorocytidine;
5′-deoxy-N⁴ -(3,5-dichlorobenzoyl)-5-fluorocytidine;
5′-deoxy-5-fluoro-N⁴ -(4-nitrobenzoyl)cytidine;
5′-deoxy-5-fluoro-N⁴ -(4-methoxycarbonylbenzoyl)cytidine;
N⁴ -(4-acetylbenzoyl)-5′-deoxy-5-fluorocytidine;
5′-deoxy-5-fluoro-N⁴ -(phenylacetyl)cytidine:
5′-deoxy-5-fluoro-N⁴ -(4-methoxyphenylacetyl)cytidine;
5′-deoxy-5-fluoro-N⁴ -nicotinoylcytidine;
5′-deoxy-5-fluoro-N⁴ -isonicotinoylcytidine;
5′-deoxy-5-fluoro-N⁴ -picolinoylcytidine;
5′-deoxy-5-fluoro-N⁴ -(2-furoyl)cytidine;
5′-deoxy-5- fluoro-N⁴ -(5-nitro-2-furoyl)cytidine;
5′-deoxy-5-fluoro-N⁴ -(2-thenoyl)cytidine;
5′-deoxy-5-fluoro-N⁴ -(5-methyl-2-thenoyl)cytidine;
5′-deoxy-5-fluoro-N⁴ -(1-methyl-2-pyrrolecarbonyl)cytidine;
5′-deoxy-5-fluoro-N⁴ -(3-indolylacetyl)cytidine;
N⁴ -(3-butenoyl)-5′-deoxy-5-fluorocytidine;
5′-O-benzoyl-5′-deoxy-5-fluorocytidine;
N⁴;3′-O-dibenzoyl-5′-deoxy-5-fluorocytidine;
5′-deoxy-N⁴ -(ethylthio)carbonyl-5-fluorocytidine;
5′-deoxy-5-fluoro-N⁴ -octadecanoylcytidine;
N⁴ -cyclopropanecarbonyl-5′-deoxy-5-fluorocytidine;
N⁴ -cyclohexanecarbonyl-5′-deoxy-5-fluorocytidine;
N⁴ -(1-adamantanecarbonyl)-5′-deoxy-5-fluorocytidine;
5′-deoxy-5-fluoro-N⁴ -(2-methoxybenzoyl)cytidine;
5′-deoxy-N⁴ -(2,4-dimethoxybenzoyl)-5-fluorocytidine;
5′-deoxy-5-fluoro-N⁴ -piperonyloylcytidine;
5′-deoxy-5-fluoro-N⁴ -(4-fluorobenzoyl)cytidine;
N⁴ -(2-chlorobenzoyl)-5′-deoxy-5-fluorocytidine;
N⁴ -(3-chlorobenzoyl)-5′-deoxy-5-fluorocytidine;
5′-deoxy-5-fluoro-N⁴ -(3-nitrobenzoyl)cytidine;
5′-deoxy-5-fluoro-N⁴ -[4-(methylthio)benzoyl]cytidine;
5′-deoxy-5-fluoro-N⁴ -(2-naphthoyl)cytidine;
5′-deoxy-5-fluoro-N⁴ -(3-furoyl)cytidine;
5′-deoxy-5-fluoro-N⁴ -(3-phenylpropionyl)cytidine;
N⁴ -cinnamoyl-5′-deoxy-5-fluorocytidine;
2′, 3′-di-O-benzoyl-5′-deoxy-5-fluorocytidine;
N⁴,2′-O,3′-O-tribenzoyl-5′-deoxy-5-fluorocytidine;
5′-deoxy-5-fluoro-N⁴ -(octyloxycarbonyl)cytidine;
N⁴ -(benzyloxycarbonyl)-5′-deoxy-5-fluorocytidine;
5′-deoxy-5-fluoro-N⁴ -formylcytidine;
5′-deoxy-5-fluoro-N⁴ -(propoxycarbonyl)cytidine;
N⁴ -(butoxycarbonyl)-5′-deoxy-5-fluorocytidine;
5′-deoxy-5-fluoro-N⁴ -(pentyloxycarbonyl)cytidine;
5′-deoxy-5-fluoro-N⁴ -(hexyloxycarbonyl)cytidine;
5′-deoxy-5-fluoro-N⁴ -(isopentyloxycarbonyl)cytidine;
5′-deoxy-5-fluoro-N⁴ -(neopentyloxycarbonyl)cytidine;
5′-deoxy-5-fluoro-N⁴ -[(1,1,2-trimethylpropoxy)carbonyl]cytidine;
5′-deoxy-N⁴ -[(3,3-dimethylbutoxy)carbonyl]-5-fluorocytidine;
5′-deoxy-5-fluoro-N⁴ -[(1-isopropyl-2-methylpropoxy)carbonyl]cytidine;
5′-deoxy-N⁴ -[(2-ethylbutoxy)carbonyl]-5-fluorocytidine;
N -[(cyclohexylmethoxy)carbonyl]-5′-deoxy-5-fluorocytidine;
5′-deoxy-5-fluoro-N⁴ -[(2-phenylethoxy)carbonyl]cytidine;
2′,3′-di-O-acetyl-5′-deoxy-5-fluoro-N⁴ -(propoxycarbonyl)cytidine;
2′,3′-di-acetyl-N⁴ -(butoxycarbonyl)-5′-deoxy-5-fluorocytidine;
2′,3′-di-benzoyl-N⁴ -(butoxycarbonyl)-5′-deoxy-5-fluorocytidine;
2′,3′-di-O-acetyl-5′-deoxy-5-fluoro-N⁴ -(pentyloxycarbonyl)cytidine;
2′,3′-di-acetyl-5′-deoxy-5-fluoro-N⁴-(isopentyloxycarbonyl)cytidine;
2′,3′-di-O-acetyl-5′-deoxy-5-fluoro-N⁴ -(hexyloxycarbonyl)-cytidine;
2′,3′-di-O-acetyl-5′-deoxy-N⁴ -[(2-ethylbutyl)oxycarbonyl]-5 -fluorocytidine;
2′,3′-di-O-acetyl-N⁴ -[(cyclohexylmethoxy)carbonyl]-5′-deoxy-5-fluorocytidine;
2′,3′-di-O-acetyl-5′-deoxy-5-fluoro-N⁴ -[(2-phenylethoxy)carbonyl]cytidine;
5′-deoxy-5-fluoro-N⁴ -(isobutoxycarbonyl)cytidine;
5′-deoxy-5-fluoro-N⁴ -[(2-propylpentyl)oxycarbonyl]cytidine;
5′-deoxy-N⁴ -[(2-ethylhexyl)oxycarbonyl]-5-fluorocytidine;
5′-deoxy-5-fluoro-N⁴ -(heptyloxycarbonyl)cytidine;
N⁴ -[(2-cyclohexylethoxy)carbonyl]-5′-deoxy-5-fluorocytidine;
N⁴ -[(3-cyclohexylpropyl)oxycarbonyl]-5′-deoxy-5-fluorocytidine;
N⁴ -(cyclohexyloxycarbonyl)-5′-deoxy-5-fluorocytidine;
5′-deoxy-5-fluoro-N⁴ -[(3-phenylpropyl)oxycarbonyl]cytidine;
5′-deoxy-5-fluoro-N⁴ -[(2-methoxyethoxy)carbonyl]cytidine, and
hydrates and salts thereof.

11. A pharmaceutical composition comprising a cytotoxic agent and a stabilized chlorite solution, wherein the cytotoxic agent comprises a prodrug of 5-fluorouracil (5-FU).

12. The pharmaceutical composition according to claim 11, wherein the stabilized chlorite solution comprises WF10.

13. The pharmaceutical composition according to claim 11 or 12, wherein the cytotoxic agent is in an oral dosage form and the stabilized chlorite solution is in the form of an intravenous dosage form or an infusion dosage form.

14. The pharmaceutical composition according to any one of claims 11 to 13, wherein the cytotoxic agent comprising a prodrug of 5-fluorouracil (5-FU) is a compound of the formula II: 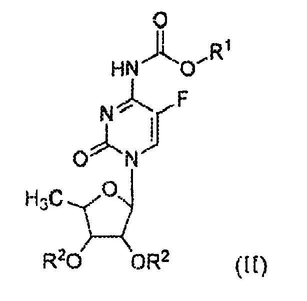 wherein R¹ is a radical of the formula -(CH₂)ₙ-Y, wherein Y is a cyclohexyl radical, a C₁-C₄alkoxy radical and, when Y is a cyclohexyl radical, n is an integer from 0 to 4 and when Y is a C₁-C₄alkoxy radical, n is an integer from 2 to 4, and R² is a hydrogen atom or C(O)O-R¹, and mixtures thereof.

15. The pharmaceutical composition according to any one of claims 11 to 13, wherein the cytotoxic agent comprising a prodrug of 5-FU is selected from the group consisting of Doxifluridine, UFT, S-1, BOF-A2, and mixtures thereof, as well as:
N⁴ -acetyl-5'-deoxy-5-fluorocytidine;
5'-deoxy-5-fluoro-N⁴ -propionylcytidine;
N⁴ -butyryl-5′-deoxy-5-fluorocytidine;
5′-deoxy-5-fluoro-N⁴ -isobutyrylcytidine;
5′-deoxy-5-fluoro-N⁴ -(2-methylbutyryl)cytidine;
5′-deoxy-N⁴ -(2-ethylbutyryl)-5-fluorocytidine;
5′-deoxy-N⁴ -(3,3-dimethylbutyryl)-5-fluorocytidine;
5′-deoxy-5-fluoro-N⁴ -pivaloylcytidine;
5′-deoxy-5-fluoro-N⁴ -valerylcytidine;
5′-deoxy-5-fluoro-N⁴ -isovalerylcytidine;
5′-deoxy-5-fluoro-N⁴ -(2-methylvaleryl)cytidine;
5′-deoxy-5-fluoro-N⁴ -(3-methylvaleryl)cytidine;
5′-deoxy-5-fluoro-N⁴ -(4-methylvaleryl)cytidine;
5′-deoxy-5-fluoro-N⁴ -hexanoylcytidine;
5′-deoxy-5-fluoro-N⁴ -heptanoylcytidine;
5′-deoxy-5-fluoro-N⁴ -octanoylcytidine;
5′-deoxy-5-fluoro-N⁴ -nonanoylcytidine;
5′-deoxy-5-fluoro-N⁴ -hexadecanoylcytidine;
N⁴ -benzoyl-5′-deoxy-5-fluorocytidine;
5′-deoxy-5-fluoro-N⁴ -(4-methylbenzoyl)cytidine;
5′-deoxy-5-fluoro-N⁴ -(3-methylbenzoyl)cytidine;
5′-deoxy-5-fluoro-N⁴ -(2-methylbenzoyl)cytidine;
5′-deoxy-N⁴ -(4-ethylbenzoyl)-5-fluorocytidine;
5′-deoxy-N⁴ -(3,4-dimethylbenzoyl)-5-fluorocytidine;
5′-deoxy-N⁴ -(3,5-dimethylbenzoyl)-5-fluorocytidine;
5′-deoxy-5-fluoro-N⁴ -(4-methoxybenzoyl)cytidine;
5′-deoxy-N⁴ -(3,4-dimethoxybenzoyl)-5-fluorocytidine;
5′-deoxy-N⁴ -(3,5-dimethoxybenzoyl)-5-fluorocytidine;
5′-deoxy-5-fluoro-N⁴ -(3,4,5-trimethoxybenzoyl)cytidine;
5′-deoxy-5-fluoro-N⁴ -(3,4,5-triethoxybenzoyl)cytidine;
5′-deoxy-N⁴ -(4-ethoxybenzoyl)-5-fluorocytidine;
5′-deoxy-5-fluoro-N⁴ -(4-propoxybenzoyl)cytidine;
5′-deoxy-N⁴ -(3,5-diethoxybenzoyl)-5-fluorocytidine;
N⁴ -(4-chlorobenzoyl)-5′-deoxy-5-fluorocytidine;
5′-deoxy-N⁴ -(3,4-dichlorobenzoyl)-5-fluorocytidine;
5′-deoxy-N⁴ -(3,5-dichlorobenzoyl)-5-fluorocytidine;
5′-deoxy-5-fluoro-N⁴ -(4-nitrobenzoyl)cytidine;
5′-deoxy-5-fluoro-N⁴ -(4-methoxycarbonylbenzoyl)cytidine;
N⁴ -(4-acetylbenzoyl)-5′-deoxy-5-fluorocytidine;
5′-deoxy-5-fluoro-N⁴ -(phenylacetyl)cytidine;
5′-deoxy-5-fluoro-N⁴ -(4-methoxyphenylacetyl)cytidine;
5′-deoxy-5-fluoro-N⁴ -nicotinoylcytidine;
5′-deoxy-5-fluoro-N⁴ -isonicotinoylcytidine;
5′-deoxy-5-fluoro-N⁴ -picolinoylcytidine;
5′-deoxy-5-fluoro-N⁴ -(2-furoyl)cytidine;
5′-deoxy-5-fluoro-N⁴ -(5-nitro-2-furoyl)cytidine;
5′-deoxy-5-fluoro-N⁴ -(2-thenoyl)cytidine;
5′-deoxy-5-fluoro-N⁴ -(5-methyl-2-thenoyl)cytidine;
5′-deoxy-5-fluoro-N⁴ -(1-methyl-2-pyrrolecarbonyl)cytidine;
5′-deoxy-5-fluoro-N⁴ -(3-indolylacetyl)cytidine;
N⁴ -(3-butenoyl)-5′-deoxy-5-fluorocytidine;
5′-O-benzoyl-5′-deoxy-5-fluorocytidine;
N⁴;3′-O-dibenzoyl-5′-deoxy-5-fluorocytidine;
5′-deoxy-N⁴ -(ethylthio)carbonyl-5-fluorocytidine;
5′-deoxy-5-fluoro-N⁴ -octadecanoylcytidine;
N⁴ -cyclopropanecarbonyl-5′-deoxy-5-fluorocytidine;
N⁴ -cyclohexanecarbonyl-5′-deoxy-5-fluorocytidine;
N⁴ -(1-adamantanecarbonyl)-5′-deoxy-5-fluorocytidine;
5′-deoxy-5-fluoro-N⁴ -(2-methoxybenzoyl)cytidine;
5′-deoxy-N⁴ -(2,4-dimethoxybenzoyl)-5-fluorocytidine;
5′-deoxy-5-fluoro-N⁴ -piperonyloylcytidine;
5′-deoxy-5-fluoro-N⁴ -(4-fluorobenzoyl)cytidine;
N⁴ -(2-chlorobenzoyl)-5′-deoxy-5-fluorocytidine;
N⁴ -(3-chlorobenzoyl)-5′-deoxy-5-fluorocytidine;
5′-deoxy-5-fluoro-N⁴ -(3-nitrobenzoyl)cytidine;
5′-deoxy-5-fluoro-N⁴ -[4-(methylthio)benzoyl]cytidine;
5′-deoxy-5-fluoro-N⁴ -(2-naphthoyl)cytidine;
5′-deoxy-5-fluoro-N⁴ -(3-furoyl)cytidine;
5′-deoxy-5-fluoro-N⁴ -(3-phenylpropionyl)cytidine;
N⁴ -cinnamoyl-5′-deoxy-5-fluorocytidine;
2′, 3′-di-O-benzoyl-5′-deoxy-5-fluorocytidine;
N⁴,2′-O,3′-O-tribenzoyl-5′-deoxy-5-fluorocytidine;
5′-deoxy-5-fluoro-N⁴ -(octyloxycarbonyl)cytidine;
N⁴ -(benzyloxycarbonyl)-5′-deoxy-5-fluorocytidine;
5′-deoxy-5-fluoro-N⁴ -formylcytidine;
5′-deoxy-5-fluoro-N⁴ -(propoxycarbonyl)cytidine;
N⁴ -(butoxycarbonyl)-5′-deoxy-5-fluorocytidine;
5′-deoxy-5-fluoro-N⁴ -(pentyloxycarbonyl)cytidine;
5′-deoxy-5-fluoro-N⁴ -(hexyloxycarbonyl)cytidine;
5′-deoxy-5-fluoro-N⁴ -(isopentyloxycarbonyl)cytidine;
5′-deoxy-5-fluoro-N⁴ -(neopentyloxycarbonyl)cytidine;
5′-deoxy-5-fluoro-N⁴ -[(1, 1,2-trimethylpropoxy)carbonyl]cytidine;
5′-deoxy-N⁴ -[(3,3-dimethylbutoxy)carbonyl]-5-fluorocytidine;
5′-deoxy-5-fluoro-N⁴ -[(1-isopropyl-2-methylpropoxy)carbonyl]cytidine;
5′-deoxy-N⁴ -[(2-ethylbutoxy)carbonyl]-5-fluorocytidine;
N -[(cyclohexylmethoxy)carbonyl]-5′-deoxy-5-fluorocytidine;
5′-deoxy-5-fluoro-N⁴ -[(2-phenylethoxy)carbonyl]cytidine;
2′,3′-di-O-acetyl-5′-deoxy-5-fluoro-N⁴ -(propoxycarbonyl)cytidine;
2′,3′-di-acetyl-N⁴ -(butoxycarbonyl)-5′-deoxy-5-fluorocytidine;
2′,3′-di-benzoyl-N⁴ -(butoxycarbonyl)-5′-deoxy-5-fluorocytidine;
2′,3′-di-O-acetyl-5′-deoxy-5-fluoro-N⁴ -(pentyloxycarbonyl)cytidine;
2′,3′-di-acetyl-5′-deoxy-5-fluoro-N⁴-(isopentyloxycarbonyl)cytidine;
2′,3′-di-O-acetyl-5′-deoxy-5-fluoro-N⁴ -(hexyloxycarbonyl)-cytidine;
2′,3′-di-O-acetyl-5′-deoxy-N⁴ -[(2-ethylbutyl)oxycarbonyl]-5 -fluorocytidine;
2′,3′-di-O-acetyl-N⁴ -[(cyclohexylmethoxy)carbonyl]-5′-deoxy-5-fluorocytidine;
2′,3′-di-O-acetyl-5′-deoxy-5-fluoro-N⁴ -[(2-phenylethoxy)carbonyl]cytidine;
5′-deoxy-5-fluoro-N⁴ -(isobutoxycarbonyl)cytidine;
5′-deoxy-5-fluoro-N⁴ -[(2-propylpentyl)oxycarbonyl]cytidine;
5′-deoxy-N⁴ -[(2-ethylhexyl)oxycarbonyl]-5-fluorocytidine;
5′-deoxy-5-fluoro-N⁴ -(heptyloxycarbonyl)cytidine;
N⁴ -[(2-cyclohexylethoxy)carbonyl]-5′-deoxy-5-fluorocytidine;
N⁴ -[(3-cyclohexylpropyl)oxycarbonyl]-5′-deoxy-5-fluorocytidine;
N⁴ -(cyclohexyloxycarbonyl)-5′-deoxy-5-fluorocytidine;
5′-deoxy-5-fluoro-N⁴ -[(3-phenylpropyl)oxycarbonyl]cytidine;
5′-deoxy-5-fluoro-N⁴ -[(2-methoxyethoxy)carbonyl]cytidine, and
hydrates and salts thereof.
